# EUROPEAN PATENT APPLICATION

(11) **EP 2 071 032 A2**
(43) Date of publication of application: **17.06.2009**
(21) Application number: 09156676.0
(22) Date of filing: 31.05.2007
(51) Int. Cl.: C12P 41/00, C07C 255/19

(54) **The use of enzymatic resolution for the preparation of intermediates of pregabalin**

(30) Priority: 31.05.2006 US 809978 P; 17.07.2006 US 831591 P; 09.08.2006 US 836730 P; 20.11.2006 US 860360 P; 10.01.2007 US 879870 P; 20.03.2007 US 919201 P; 23.04.2007 US 926059 P
(62) Divisional of application: 07795616.7
(71) Applicant: Teva Pharmaceutical Industries Ltd, 49131 Petah Tiqva (IL)
(72) Inventor: Hedvati, Lilach, 37845 Doar Na Hefer (IL); Fishman, Ayelet, 34814 Haifa (IL)
(74) Representative: Eder, Michael

(57) **Abstract**

Provided is the use of enzymatic resolution for the preparation of intermediates of prcgabalin, including (3S)-cyano-5-methylhcxanoic acid and salts thereof and R-(-)-3-(carbamoylmethyl)-5-methylhexanoic acid and salts thereof.

## Description

### Cross-Reference to Related Applications

This application claims the benefit of priority to U.S. provisional application Serial Nos. 60/809,978, filed May 31, 2006; 60/831,591, filed July 17, 2006; 60/836,730, filed August 9, 2006; 60/860,360, filed November 20, 2006; 60/879,870, filed January 10, 2007; 60/919,201, filed March 20, 2007; and 60/926,059, filed April 23, 2007, hereby incorporated by reference.

### Field of the Invention

The invention encompasses the use of enzymatic resolution for the preparation of intermediates of pregabalin, including (3S)-cyano-5-methylhexanoic acid and salts thereof and R-(+)-3-(carbamoylmethyl)-5-methylhexanoic acid and salts thereof.

### Background of the Invention

(S)-Pregabalin, (S)-(+)-3-(aminomethyl)-5-methylhexanoic acid, a compound having the chemical structure, 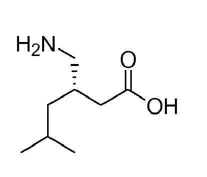 is also known as γ-amino butyric acid or (S)-3-isobutyl GABA. (S)-Pregabalin has been found to activate GAD (L-glutamic acid decarboxylase). (S)-Pregabalin has a dose dependent protective effect on-seizure, and is a CNS-active compound. (S)-Pregabalin is useful in anticonvulsant therapy, due to its activation of GAD, promoting the production of GABA, one of the brain's major inhibitory neurotransmitters, which is released at 30 percent of the brains synapses. (S)-Pregabalin has analgesic, anticonvulsant, and anxiolytic activity.

A non-asymmetric preparation of (S)-pregabalin is disclosed in U.S. Patent No. 5,616,793, and in DRUGS OF THE FUTURE, 24 (8), 862-870 (1999) and is performed by obtaining the intermediate (±)3-(Carbamoylmethyl)-5-methylhexanoic acid ("CMH" or "CMH-racemate"), which is then optically resolved to give R-(+)3-(carbamoylmethyl)-5-methylhexanoic acid ("R-CMH"), which is then converted to (S)-pregabalin, as described in the following Scheme 1.

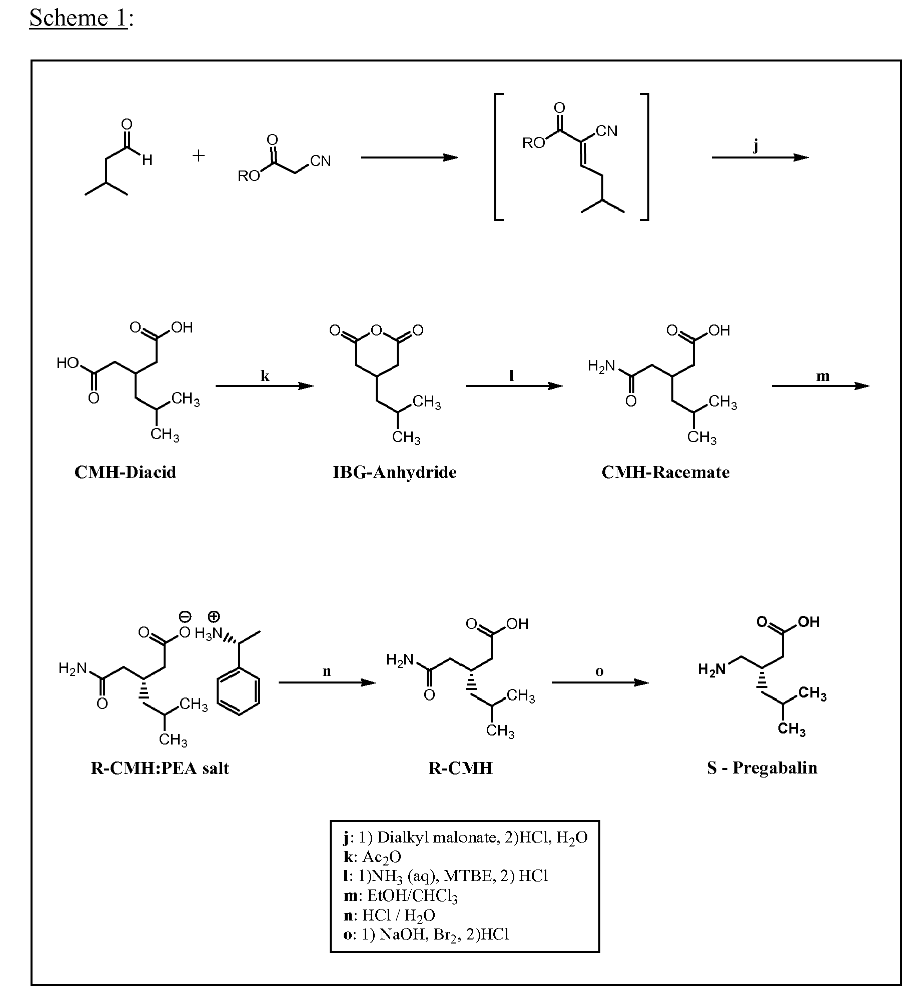

Another non-asymmetric process is reported in U.S. Patent No. 5,637,767, wherein the preparation of (S)-pregabalin is accomplished by hydrolysis and decarboxylation of product II of the following structure: 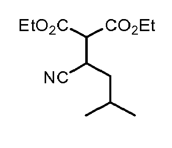 to give 3-cyano-5-methylhexanoic acid ethyl ester ("II-CN-monoester") of the following structure: 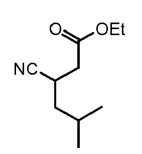 which further undergoes hydrogenation to obtain racemic pregabalin ("PRG-racemate") of the following structure: 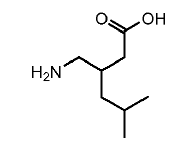 followed by optical resolution to obtain the S-enantiomer of pregabalin.

U.S. Publication No. 2005/0283023 describes the preparation of the intermediate (3S)-cyano-5-methylhexanoic acid ("(S)-pregabalin nitrile" or "S-PRG-nitrile") by enzymatic kinetic resolution of a cyano-dialkylester, followed by converting the resolved enantiomer to various intermediates, which are then converted to S-PRG-nitrile.

There is a need for additional processes for the preparation of intermediates of pregabalin, especially, S-PRG-nitrile and salts thereof and R-CMH and salts thereof.

### Summary of the Invention

In one embodiment, the invention encompasses a process for preparing a pregabalin intermediate of the following formula I 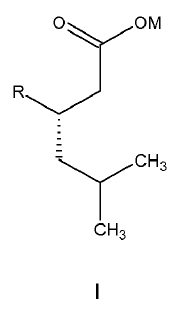 comprising enzymatically hydrolyzing an ester of the following formula II 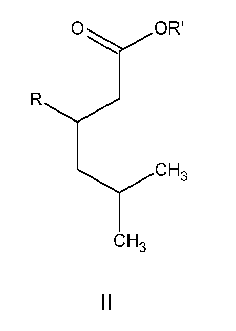 in the presence of a buffer and optionally a base, wherein R is CH₂CONR"₂, CH₂CO₂R' or CN; R' is a C₁₋₆ hydrocarbyl; R" is hydrogen or a C₁₋₆ hydrocarbyl; and M is a metal.

In another embodiment, the invention encompasses a process for preparing a pregabalin intermediate of the following formula I 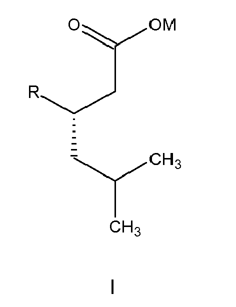 comprising: a) combining an ester of the following formula II 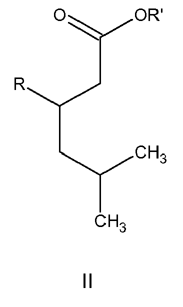 a hydrolase, a buffer, and optionally a base to obtain a mixture; and b) maintaining the mixture at a temperature of about 5°C to about 60°C to obtain the pregabalin intermediate of formula I, wherein R is CH₂CONR"₂, CH₂CO₂R' or CN; R' is a C₁₋₆ hydrocarbyl; R" is hydrogen or a C₁₋₆ hydrocarbyl; and M is a metal.

In another embodiment, the invention encompasses a process for preparing a pregabalin intermediate of the following formula I-CN 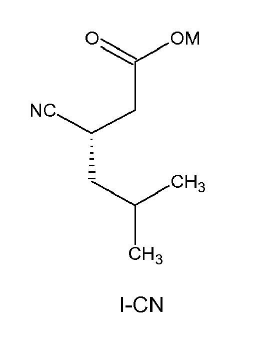 comprising:
a) decarboxylating a (±)-2-carboxyalkyl-3-cyano-5-methyl hexanoic acid alkyl ester of the following formula 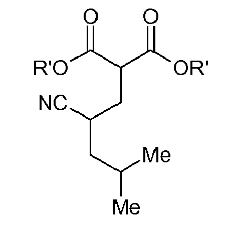 by combining it with an alkaline hydroxide to obtain an ester of the following formula II-CN-monoester; 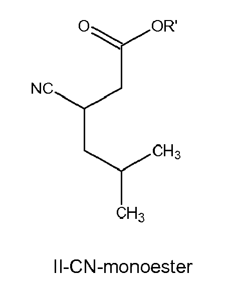
b) isolating the obtained compound of formula II-CN-monoester; c) combining the compound of the formula II-CN-monoester, a hydrolase, a buffer, and optionally a base to obtain a mixture; c) maintaining the mixture at a temperature of about 5°C to about 60°C to obtain a compound of the following formula I-CN; 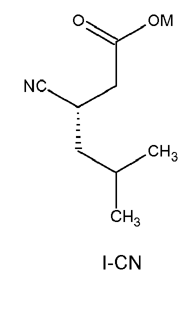 wherein R' is a C₁₋₆ hydrocarbyl; and M is a metal.

In another embodiment, the invention encompasses a process for preparing a pregabalin intermediate of the following formula I-acid 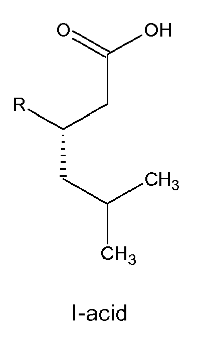 comprising enzymatically esterifying a compound of the following formula III, 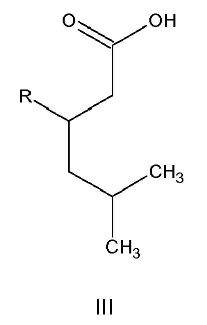 wherein R is CH₂CONR"₂, CH₂CO₂R' or CN; R' is a C₁₋₆ hydrocarbyl; and R" is a hydrogen or a C₁₋₆ hydrocarbyl.

In another embodiment, the invention encompasses a process for preparing a pregabalin intermediate of the following formula I-acid 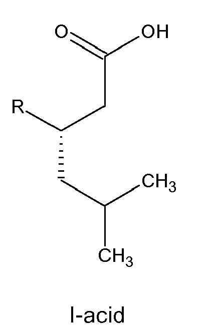 comprising combining a compound of the following formula III, 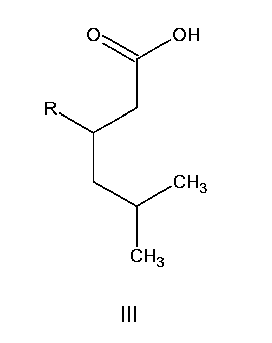 an alcohol or an ester, and an enzyme to obtain the pregabalin intermediate of formula I-acid, wherein R is CH₂CONR"₂, CH₂CO₂R' or CN; R' is a C₁₋₆ hydrocarbyl; and R" is a hydrogen or C₁₋₆ hydrocarbyl.

In another embodiment, the invention encompasses a process for preparing (S)-pregabalin comprising preparing the pregabalin intermediate of formula I or formula I-acid by any of the above-described processes, and converting the pregabalin intermediate into (S)-pregabalin.

### Detailed Description of the Invention

As used herein, unless otherwise defined, the term "PRG" refers to pregabalin.

As used herein, unless otherwise defined, the term "racemate" refers to a mixture that contains an equal amount of enantiomers.

The invention encompasses processes for preparing pregabalin intermediates through enzymatic resolution, wherein the process is a kinetic resolution process. Preferably, the invention encompasses processes for preparing the pregablin intermediates S-PRG-nitrile and salts thereof and R-CMH and salts thereof through enzymatic resolution. The processes can be illustrated by the following general Scheme 2. 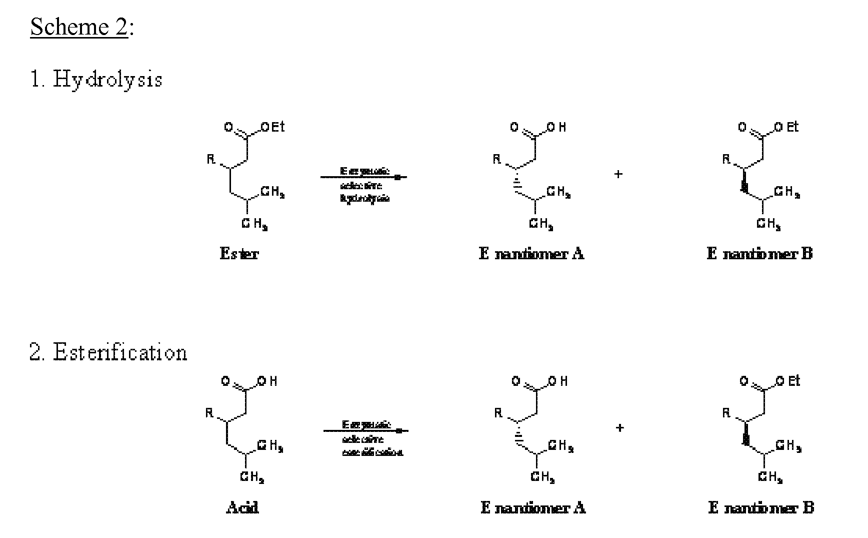 where the resolution, which is an enzymatic resolution, can be done by either hydrolysis or esterification.

It is well known that enzymes are very specific in their functions due to the amino acids present in their active site. Also, enzymes are chiral and have asymmetric binding sites; this asymmetry leads to enzyme stereospecificity, which results in its favor to bind one enantiomer over the other. In addition, enzymes may be recycled due to the fact that their structure does not change during the reaction, thus, the use of enzymes makes the processing easier, because the isolation of the enzyme from the reaction mixture is simple.

The benefit of performing the optical resolution on these intermediates instead of on pregabalin racemate is significant, since the undesired enantiomer can be easily recycled while the recycling of the undesired enantiomer of pregabalin is very difficult.

In one embodiment, the invention encompasses a process for preparing a pregabalin intermediate of formula I, which may be illustrated by the following Scheme 3. 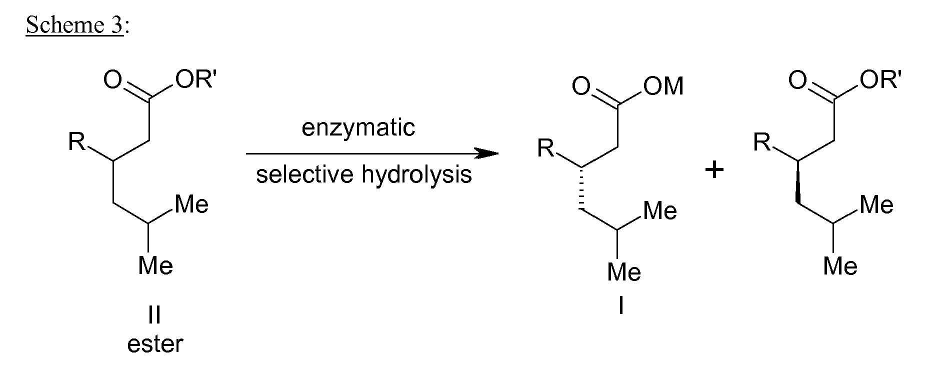 wherein R is CH₂CONR"₂ , CH₂CO₂R' or CN; R' is a C₁₋₆ hydrocarbyl; R" is a hydrogen or C₁₋₆ hydrocarbyl; R' is a C₁₋₆ hydrocarbyl; and M is a metal, wherein the metal is provided by the buffer or the base. Preferably, the CH₂CONR"₂ is a CH₂CONH₂. Preferably, the CH₂CO₂R' is CH₂CO₂Me, CH₂CO₂Et, CH₂CO₂- vinyl, CH₂CO₂- propyl, or CH₂CO₂- isopropyl, and more preferably CH₂CO₂Me, CH₂CO₂Et, or CH₂CO₂- vinyl. Most preferably, R is either CN or a CH₂CONH₂. Preferably, the C₁₋₆ hydrocarbyl is a C₁₋₃ hydrocarbyl and more preferably either ethyl or methyl. Preferably, M is an alkali metal and more preferably either potassium or sodium.

The process comprises: (a) combining the ester of formula II with a hydrolase, a buffer, and optionally a base to obtain a mixture; and (b) maintaining the mixture at a temperature of about 5°C to about 60°C to obtain the pregabalin intermediate of formula I, wherein the metal is provided by the buffer or the base. The buffer and the base preferably contain the same metal.

The process employs a hydrolase, i.e., an enzyme that performs a stereoselective hydrolysis reaction by reacting with only one enantiomer of the ester of formula II to provide the chiral pregabalin intermediate of formula I. Thus, the chiral pregabalin intermediate of formula I can be selectively produced via kinetic resolution.

When R is CN and R' is ethyl, the compound of formula II is (±)-3-cyano-5-methylhexanoic acid-ethyl ester ("II-CN-monoester") of the following structure: 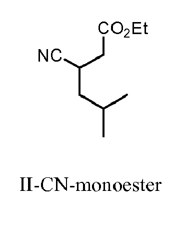 and when R is CN and M is Na, the compound of formula I is S-PRG-nitrile sodium ("I-CN-Na") of the following structure:

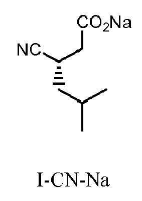

When R is CH₂CONH₂ and R' is ethyl, the compound of formula II is (±)3-(carbamoylmethyl)-5-methylhexanoic ethyl ester ("II-amide-monoester") of the following structure: 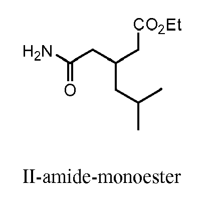 and when R is CH₂CONH₂, and M is Na, the compound of formula I is R-CMH-sodium ("I-amide-Na") of the following structure:

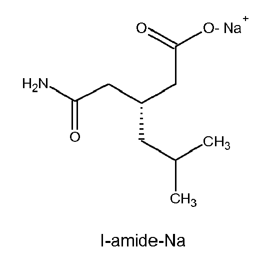

Preferably, the hydrolase is either an esterase, lipase or protease. Preferably, the esterase is selected from the group consisting of Esterase PF2 recombinant in *E.Coli,* Esterase BS1 recombinant in *E.Coli,* Esterase BS2 recombinant in *E. Coli,* Esterase BS2 CLEA recombinant in *E. Coli,* Esterase BS3 recombinant in *E. Coli,* Esterase BS4 recombinant in *E. Coli,* Esterase PL from porcine liver, Esterase SD recombinant in *E. Coli,* Esterase RO, and Esterase TL recombinant in *Aspergillus oryzae.*

Preferably, the lipase is selected from the group consisting of Lipase from *Thermomyces lanuginosus,* Lipase P2 from *Pseudomonas cepacia* Lipase PS from *Pseudomonas stutzeri,* Lipase RS from *Rhizopus sp.,* Lipase PF from *Pseudomonas fluorescens,* Lipase PC from *Penicillium camenbertii,* Lipase P1 from *Pseudomonas cepacia,* Lipase AN from *Aspergillus niger,* Lipase A from *Achromobacter sp.,* Lipase AS1 from *Alcaligenes sp.,* Lipase AS2 *Alcaligenes sp,* Lipase C2 from *Candida cylindracea,* Lipase C1 from *Candida cylindracea,* Lipase lipozym TL IM, Lipase lipozym TL 100L, *Candida antarctica* lipase B (CALB), CHIRAZYME E-1 pig liver esterase, Lipase from *Pseudomonas sp.* L-6, *Candida antarctica* lipase A (CALA), *Candida rugosa* lipase (L-3), Pancreatic lipase USP Grade, Lipase QLM, and Lipase TL.

Preferably, the protease is chymotrypsin.

More preferably, the hydrolase is CALB, CHIRAZYME E-1 pig liver esterase, Esterase BS3 recombinant in *E.Coli,* or Esterase PL from porcine liver.

Typically, enzymes are used in a combination with a buffer. The buffer provides a pH suitable for the enzyme activity. Preferably, the buffer is present in an amount sufficient to provide a pH of about 6 to about 9, more preferably about 6.5 to about 8, and most preferably about 7.

Typically, the base is added to help control the pH of the combination of step a). The base may be a hydroxide, carbonate, or hydrogen carbonate of an alkali metal or alkaline earth metal hydroxide. Preferably, the base is a hydroxide, carbonate or hydrogen carbonate of an alkali metal. More preferably, the base is an alkali metal hydroxide, and most preferably, either NaOH or KOH.

Typically, the hydrolase, the buffer, and optionally the base, are combined first, followed by addition of the ester of formula II to obtain the mixture. The ester of formula II can be racemate or a mixture of the enantiomers in any ratio. A co-solvent may be added to the buffer to facilitate solubilization of the substrate. Suitable co-solvents include, but are not limited to sulfoxides, amides, alcohols, ketones and nitriles. Preferably, the sulfoxide is a C₂₋₄ sulfoxide, and more preferably dimethylsulfoxide ("DMSO"). Preferably, the amide is a C₃₋₆ amide, and more preferably dimethylformamide ("DMF"). Preferably, the alcohol is a C₁₋₆ alcohol, and more preferably isopropyl alcohol. Preferably, the ketone is a C₂₋₆ ketone, and more preferably acetone. Preferably, the nitrile is a C₁₋₅ nitrile, and more preferably acetonitrile.

Preferably, the mixture is maintained, while stirring, to obtain the pregabalin intermediate of formula I. More preferably, the mixture is maintained for about 8 to about 32 hours, and even more preferably for about 24 hours. Preferably, the mixture is stirred at a temperature of about 20°C to about 27°C, and more preferably at a temperature of about 22°C to about 25°C.

The pregabalin intermediate of formula I may be recovered by any method known to one of ordinary skill in the art. Such methods include, but are not limited to, extraction.

The pregabalin intermediate of formula I thus prepared may optionally be converted into an intermediate of the following formula I-acid 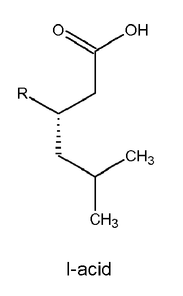 wherein R is CH₂CONR"₂ , CH₂CO₂R' or CN; R' is a C₁₋₆ hydrocarbyl; R" is a hydrogen or C₁₋₆ hydrocarbyl; R' is a C₁₋₆ hydrocarbyl. The conversion may be performed by a process comprising combining the intermediate of formula I with an inorganic acid selected from the group consisting of HBr, H₂SO₄, H₃PO₄, and HCl. Preferably, the inorganic acid is HCl.

The pregabalin intermediate of formula I or formula I-acid thus prepared may be converted into (S)-pregabalin. The conversion may be performed, for example, according to the process disclosed in U.S. Publication No. 2007/0073085 or in U.S. Patent No. 5,637,767, both of which are hereby incorporated by reference.

In one preferred embodiment, when R is CN, the ester of formula II ("II-CN-monoester") may be prepared by decarboxylating a (±)-2-carboxyalkyl-3-cyano-5-methyl hexanoic acid alkyl ester ("PRG-Nitrile diester"). This process may be illustrated by the following Scheme 4. 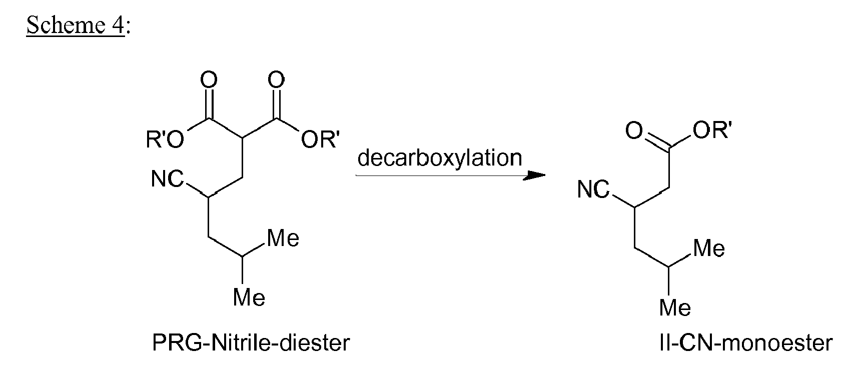 where R' is a C₁₋₆ hydrocarbyl. Preferably, the C₁₋₆ hydrocarbyl is a C₁₋₃ hydrocarbyl, and more preferably either ethyl or methyl.

The process comprises: (a) combining PRG-Nitrile-diester and an alkaline hydroxide to obtain a mixture having II-CN-monoester; and (b) isolating the II-CN-monoester from the mixture.

Typically, the PRG-Nitrile-diester and the alkaline hydroxide are combined in the presence of a solvent. Preferably, the solvent is selected from the group consisting of water, a polar organic solvent, and mixtures thereof. Preferably, the polar organic solvent is a polar protic organic solvent. Preferably, the polar protic organic solvent is a C₁-₅ alcohol. Preferably, the C₁₋₅ alcohol is a C₁₋₃ alcohol, and more preferably a C₁₋₂ alcohol. Preferably, the C₁₋₂ alcohol is either methanol or ethanol. 1.

Preferably, the alkaline hydroxide is potassium hydroxide.

Preferably, the combination of PRG-nitrile-diester and alkaline hydroxide is heated to decarboxylate the PRG-nitrile-diester and obtain the mixture having the II-CN-monoester.. Preferably, the combination is heated to a temperature of about 60°C to about 180°C, and more preferably to about 80°C to about 140°C. Preferably, the combination is heated for about 8 to about 24 hours.

The II-CN-monoester thus obtained may be isolated by any method known to one of ordinary skill in the art. Such methods include, but are not limited to, extracting the II-CN-monoester from the mixture with a solvent and evaporating the solvent. Preferably, the II-CN-monoester is recovered by a process comprising: cooling the mixture; removing the solvent; adding a solvent selected from a group consisting of dichloromethane ("DCM"), ether, ethyl acetate, and acetonitrile to obtain an organic phase; extracting the organic phase with water, and removing the solvent from the organic phase to obtain a residue of the II-CN-monoester. Preferably, the mixture is cooled at a temperature of about 40°C to about 10°C. The solvent may be removed by evaporation under vacuum. Preferably, the solvent is DCM.

The isolated II-CN-monoester is a mixture of enantiomers of the following structure: 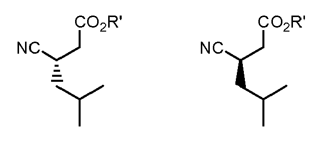 The mixture may contain the enantiomers in any ratio. Preferably, the mixture is a racemic mixture of the enantiomers.

Optionally, the isolated residue of the II-CN-monoester may be purified. Preferably, the residue is purified by distillation. Preferably, the distillation is performed at a pressure of about 1 to about 10 mm Hg, and at a temperature of about 80°C to about 100°C.

The II-CN-monoester may then be converted to the compound of formula I-CN, as illustrated by the following Scheme 5. 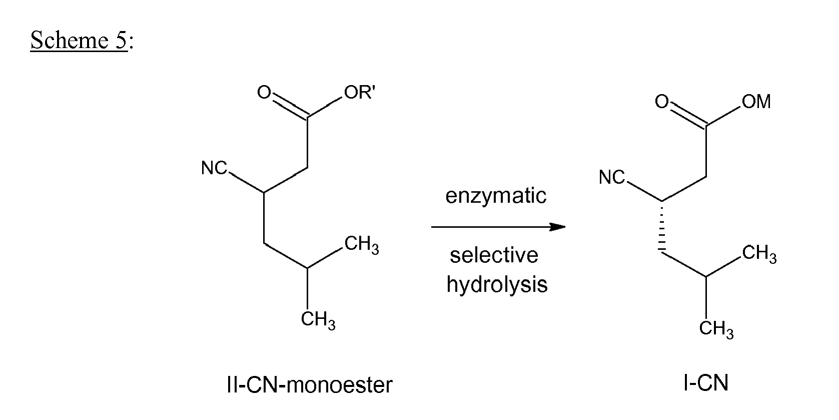 The conversion is performed by a process comprising combining the compound of formula II-CN-monoester, a hydrolase, a buffer, and optionally a base to obtain a mixture; and maintaining the mixture at a temperature of about 5°C to about 60°C, as described above.

The I-CN thus obtained may be converted into (S)-pregabalin. The conversion may be performed, for example, according to the process disclosed in U.S. Patent No. 5,637,767.

In another embodiment, the invention encompasses a process for preparing a pregabalin intermediate of formula I-acid, which may be illustrated by the following Scheme 6. 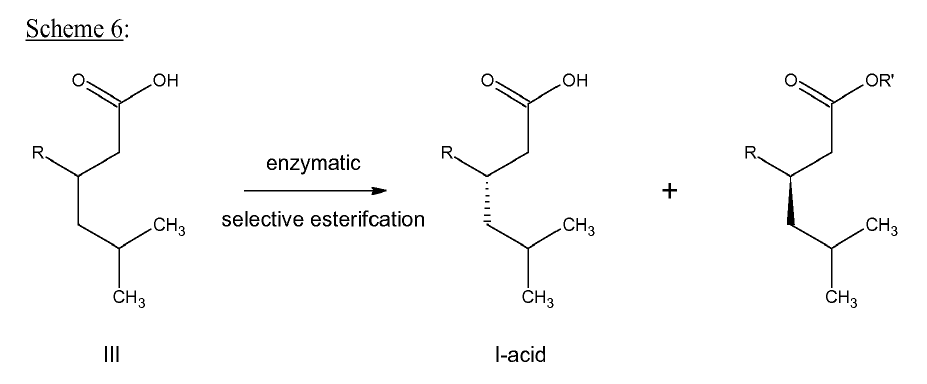 wherein R is CH₂CONR"₂, CH₂CO₂R' or CN; R' is a C₁₋₆ hydrocarbyl; and R" is a hydrogen or C₁₋₆ hydrocarbyl. Preferably, the CH₂CONR"₂ is a CH₂CONH₂. Preferably, the CH₂CO₂R' is CH₂CO₂Me, CH₂CO₂Et, CH₂CO₂- vinyl, CH₂CO₂- propyl, or CH₂CO₂-isopropyl, and more preferably CH₂CO₂Me, CH₂CO₂Et, or CH₂CO₂- vinyl. Most preferably, R is either CN or a CH₂CONH₂. Preferably, the C₁₋₆ hydrocarbyl is a C₁₋₃ hydrocarbyl and more preferably either ethyl or methyl.

The process comprises: combining the compound of formula III, an alcohol or an ester, and an enzyme to obtain the pregabalin intermediate of formula I-acid.

When R is CN, the compound of formula III is (±)-3-cyan-5-methylhexanoic acid ("III-CN-acid") of the following structure. 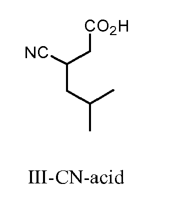 and the compound of formula I-acid is S-PRG-nitrile ("I-CN-acid") of the following structure.

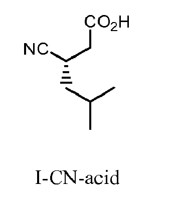

When R is CH₂CONH₂, the compound of formula III is CMH of ("III-amide-acid") of the following structure. 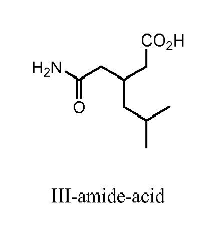 and the compound of formula I-acid is R-CMH ("I-amide-acid") of the following structure.

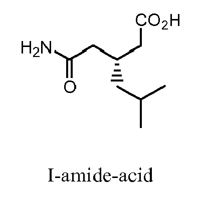

Typically, the compound of formula III, the alcohol or ester, and the enzyme are combined in the presence of a solvent. Preferably, the solvent is an organic solvent. Preferably, the organic solvent is selected from the group consisting of aromatic hydrocarbons, ethers, ketones, nitriles, chlorinated hydrocarbons, amides, and mixtures thereof. Preferably, the aromatic hydrocarbon is a C₆₋₈ aromatic hydrocarbon, and more preferably toluene. A preferred ether is a C₂₋₈ linear, branched or cyclic ether. A more preferred C₂₋₈ linear, branched or cyclic ether is a C₂₋₆ linear, branched or cyclic ether, and a most preferred C₂₋₈ linear, branched or cyclic ester is diisopropylether, methyl-tertbutylether, or tetrahydrofuran. Preferably, the ketone is a C₂₋₈ ketone. A more preferred C₂₋₈ ketone is C₂₋₄ ketone, and a most preferred C₂₋₈ ketone is methyl-ethyl ketone, methyl-isobutyl ketone, or acetone. Preferably, the nitrile is a C₂₋₅ nitrile, and more preferably acetonitrile. Preferably, the chlorinated hydrocarbon is a C₁₋₄ chlorinated hydrocarbon, and more preferably, dichloromethane or tetrachloromethane. Preferably, the amide is a C₃₋₆ amide, and more preferably dimethylformamide. The most preferred organic solvent is toluene, methyl-tertbutylether or a mixture of toluene and acetone.

Typically, the starting compound of formula III is a mixture of enantiomers of the following structure: 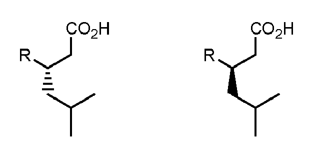 The mixture may contain the enantiomers in any ratio. Preferably, the mixture is a racemic mixture of the enantiomers.

The enzyme is any enzyme that is suitable for esterification or transesterification reactions. Preferably, the enzyme is a hydrolase, and more preferably an esterase, lipase or protease. Preferably, the enzymes that can be used in this reaction are as described above.

Preferably, the alcohol is selected from a group consisting of: methanol, ethanol, propanol and n-butanol, and mixtures thereof. Preferably, the ester is vinyl acetate or vinyl butyrate.

Typically, the combination of the compound of formula III, the alcohol or ester, and the enzyme is maintained at a temperature of about 5°C to about 70°C to obtain the pregabalin intermediate of formula I-acid. Preferably, the combination is maintained at a temperature of about 25°C to about 37°C. Preferably, the combination is maintained for about 2 to about 96 hours, and more preferably for about 48 hours.

The ester or alcohol can be used in a stoichiometric amount vs. the starting acid of formula III, or can be used in excess, thus acting also as a solvent. When a stoichiometric amount is used, the ester or alcohol and the compound of formula III are combined in a ratio of about 1 mole of ester or alcohol to about 1 mole of the compound of formula III. Preferably, the ester of alcohol is used in excess. Preferably, the molar ratio of the alcohol or the ester to the starting acid of formula III is of about 3 to about 10, respectively. Preferably, the ratio is of about 2:1 1 to about 3:1, respectively.

During this time, the enzyme binds in a selective manner to the S-enantiomer of the compound formula I-acid, thereby promoting esterification of the S-enantiomer over the R-enantiomer.

The pregabalin intermediate of formula I-acid may be recovered by any method known to one of ordinary skill in the art. Preferably, the pregabalin intermediate of formula I-acid is recovered by filtration; extraction of the filtrate with a base to obtain the salt of the compound of formula I-acid; adding an acid to convert the salt to the free acid, the compound of formula I-acid, and filtering it. The base may be an inorganic base, preferably, an aqueous solution of an inorganic base. Preferably, the inorganic base is sodium hydroxide. Preferably, prior to adding the acid, the aqueous phase is extracted with an organic solvent. Preferably, the organic solvent is toluene. The acid may be a mineral acid. Preferably, the mineral acid is HCl, HBr, H₂SO₄, or H₃PO₄. Preferably, the acid is added to the aqueous phase to provide a pH of about 1 to about 4, and more preferably about 2 to about 3.

The pregabalin intermediate of formula I-acid thus prepared may be converted into (S)-pregabalin. The conversion may be performed, for example, according to the process disclosed in U.S. Publication No. 2007/073085 or in US Patent No. 5,637,767.

Having described the invention with reference to certain preferred embodiments, other embodiments will become apparent to one skilled in the art from consideration of the specification. The invention is further defined by reference to the following examples. It will be apparent to those skilled in the art that many modifications, both to materials and methods, may be practiced without departing from the scope of the invention.

### Examples

### Enzymatic Hydrolysis

### Example 1: Enzymatic hydrolysis of (±)3-(Carbamoylmethyl)-5-methylhexanoic ethyl ester (CMH-ethyl ester)

A reactor (1.5 1) is charged with buffer (250 ml), water (200 ml), and Lipase. After a clear solution is obtained, CMH-ethyl ester is added to the solution. The resulting mixture is stirred for 24 hours at room temperature. NaOH (30% solution) is added to the mixture to adjust the pH to 7. The organic phase is then separated, and the aqueous phase is extracted with toluene twice (2x78 g). The aqueous phase contains (3S)-Cyano-5-methylhexanoic acid sodium salt, and is used in the enzymatic esterification step.

### Example 2: Enzymatic hydrolysis of CMH-ethyl ester

A vial (20ml), equipped with magnetic stirrer, was charged with buffer (3ml), CMH-ethyl ester (215mg), and Lipase from *Thermomyces lanuginosus* (100mg). The resulting mixture was stirred for 2 days at room temperature. The presence of CMH was analyzed by HPLC.

### Example 3: Enzymatic hydrolysis of CMH-ethyl ester

A vial (20ml), equipped with magnetic stirrer, was charged with buffer (3ml), CMH-ethyl ester (215mg), and Lipase from *Thermomyces lanuginosus* (100mg). The resulting mixture was stirred for 4 days at 37°C. The presence of CMH was analyzed by HPLC.

### Example 4: Enzymatic hydrolysis of CMH-ethyl ester

A vial (20ml), equipped with magnetic stirrer, was charged with buffer (3ml), CMH-ethyl ester (215mg), Lipase AN from *Aspergillus niger,* (20mg) and tetrahydrofuran (0.6ml, 20%). The mixture was stirred for 3 days at room temperature. The presence of CMH was analyzed by HPLC.

### Examples 5-10: Enzymatic hydrolysis of CMH-ethyl ester

The procedure of example 4 was repeated substituting the Lipase AN from *Aspergillus niger* with each of the following enzymes: Lipase A from *Achromobacter sp.* (example 5); Lipase AS 1 from *Alcaligenes sp* (example 6); Lipase C2 from *Candida cylindracea* (example 7); Lipase AS2 *Alcaligenes sp* (example 8); Lipase C 1 from *Candida cylindracea* (example 9); and Lipase C2 from *Candida cylindracea* (example 10).

### Example 11: Enzymatic hydrolysis of CMH-ethyl ester

A vial (20ml), equipped with magnetic stirrer, was charged with buffer (3ml), CMH-ethyl ester (215mg), and Lipase C2 from *Candida cylindracea* (20mg). The mixture was stirred for ∼2-3 days at room temperature. The resulting solution was extracted with ethyl acetate (6ml). The organic layer was separated and evaporated until dryness. The presence of CMH was analyzed by HPLC.

### Example 13: Enzymatic hydrolysis of CMH-ethyl ester

The procedure of example 12 was repeated substituting the Lipase C2 from *Candida cylindracea* with Lipase AS1 from *Alcaligenes sp* (example 13).

### Example 14: Enzymatic hydrolysis of CMH-ethyl ester

A vial (20ml), equipped with magnetic stirrer, was charged with buffer (3ml), CMH-ethyl ester (215mg), Lipase from *Thermomyces lanuginosus* (100mg) and tetrahydrofuran (0.6ml, 20%). The mixture was stirred for 4 days at 37°C. Toluene (6ml) was added to the mixture to form a biphasic mixture. The organic phase was separated and evaporated until dryness. The presence of CMH was analyzed by HPLC.

### Example 15: Enzymatic hydrolysis of CMH-ethyl ester

A vial (20ml), equipped with magnetic stirrer, was charged with buffer (3ml), CMH-ethyl ester (215mg), Lipase from *Thermomyces lanuginosus* (100mg) and dimethyl sulfoxide (0.6ml, 20%). The resulting mixture was stirred for 4 days at 37°C. Toluene (6ml) was added to the mixture to form a biphasic mixture. The organic phase was separated and evaporated until dryness. The presence of CMH was analyzed by HPLC.

### Example 16: Enzymatic hydrolysis of CMH-ethyl ester

A vial (20ml), equipped with magnetic stirrer, was charged with Buffer (0.1M of K₂HPO₄, pH=7, 9ml), CMH-ethyl ester (600mg) and CHIRAZYME E-1 (151mg). The mixture (yellow-brown) was stirred for 3 days at room temperature. The resulting solution was extracted with toluene. The aqueous layer was evaporated and R-CMH was obtained (Optical purity - 60%).

### Example 17: Enzymatic hydrolysis of CMH-ethyl ester

A vial (20ml), equipped with magnetic stirrer, was charged with buffer (0.1M of K₂HPO₄, pH=7,9ml), CMH-ethyl ester (613.4mg), and CAL B (153mg). The mixture (white slurry) was stirred for 3 days at room temperature. The resulting solution was extracted with toluene. The aqueous layer was evaporated and R-CMH was obtained (Optical purity - 98%)

### Example 18: Enzymatic hydrolysis of CMH-ethyl ester

A vial (20ml), equipped with magnetic stirrer, was charged with buffer (0.1M of K₂HPO₄, pH=7, 9ml), CMH-ethyl ester (ge-1349-3, 606.21mg), and the Esterase BS3 recombinant in *E.Coli* (156.12mg). The yellow emulsion was stirred for 3 days at room temperature. The resulting solution was extracted with toluene. The aqueous layer was evaporated and S-CMH was obtained (Optical purity - 79%)

### Example 19: Enzymatic hydrolysis of CMH-ethyl ester

A vial (20ml), equipped with magnetic stirrer, was charged with buffer (0.1M of K₂HPO₄, pH=7,9ml), CMH-ethyl ester (606.21mg) and the Esterase PL from porcine liver (150mg). The brown emulsion was stirred for 3 days at room temperature. The resulting solution was extracted with toluene. The aqueous layer was evaporated and R-CMH was obtained (Optical purity - 68%).

### Example 20: Enzymatic hydrolysis of 3-Cyano-5-methylhexanoic acid -ethyl ester

A reactor (1.5 1) is charged with buffer (250 ml), water (200 ml), and hydrolase. After a clear solution is obtained, 3-Cyano-5-methylhexanoic acid ethyl ester is added. The resulting mixture is stirred for 24 hours at room temperature. NaOH (30% solution) is added to the mixture to adjust the pH to 7. The organic phase is separated, and the aqueous phase is extracted with toluene twice (2x78 g). The aqueous phase contains (3S)-Cyano-5-methylhexanoic acid sodium salt, and is used in the enzymatic esterification step.

### Example 21: Enzymatic hydrolysis of 3-Cyano-5-methylhexanoic acid -ethyl ester

A vial (20ml), equipped with magnetic stirrer, was charged with Buffer (2.5ml), cyano ethyl ester (183mg) Pancrelipase USP Grade (20mg) and tetrahydrofuran (0.5ml, 20%). The mixture is stirred for 4 days at room temperature. The presence of CMH was analyzed by HPLC.

### Examples 22-24: Enzymatic hydrolysis of 3-Cyano-5-methylhexanoic acid -ethyl ester

The procedure of example 22 was repeated substituting the Pancrelipase USP Grade with each of the following enzymes: Lipase TL Meito sangyo (example 22); Lipase QLM (example 23); and Lipase from *Thermomyces lanuginosus* (example 24).

### Example 25: Enzymatic hydrolysis of 3-Cyano-5-methylhexanoic acid -ethyl ester

A vial (20ml), equipped with magnetic stirrer, was charged with buffer (2.5ml), cyano ethyl ester (186mg) and Lipase from *Thermomyces lanuginosus* (100mg). The resulting mixture was stirred for 3 days at room temperature. The presence of CMH was analyzed by HPLC.

### Example 26: Decarboxylation of (±)-2-Carboxyethyl-3-cyano-5-methyl hexanoic acid ethyl ester

A reactor (0.5 1) was loaded with ethanol (225 ml) and KOH (31.8 g). The mixture was cooled to room temperature and (±)-2-Carboxyethyl-3-cyano-5-methyl hexanoic acid ethyl ester (150 g) was added. The mixture was heated to reflux for 21 hours, and then cooled to room temperature. The solvent was evaporated under vacuum, and the residue was dissolved in CH₂Cl₂ (600 ml). The solution was extracted with water (600 ml), and the organic phase was separated and evaporated. The product (±)-3-Cyano-5-methylhexanoic acid ethyl was obtained as yellow oil (77 g). After purification by distillation (80-100°C, 1 mm Hg) 57 g of yellowish oil were obtained.

### Enzymatic Esterification

### Example 27: Enzymatic esterification of CMH

A reactor (1.5L) is charged with toluene (250 ml), vinyl acetate (300 mmol), enzyme (2g) and CMH-Racemate (100 mmol). The mixture is stirred for 48h at room temperature. The solution is filtered and the filtrate is extracted with NaOH (30% solution). The organic phase is separated and the aqueous phase is extracted with toluene. The aqueous phase is acidified to pH 2 to precipitate R-CMH, and the R-CMH is filtered and washed with water.

### Example 28: Enzymatic esterification of CMH

A vial (20ml), equipped with magnetic stirrer, was charged with toluene (3ml), butanol (0.25ml, 2.76mmol), Pancrelipase USP Grade (20mg) and CMH-Racemate (187mg, 0.092mmol). The resulting mixture was stirred for 3 days at room temperature. A sample was taken from the mixture (0.5ml) and dried with N₂ flow. The presence of CMH-ester in the sample was analyzed by HPLC.

### Example 29-31: Enzymatic esterification of CMH

The procedure of example 28 was repeated substituting the Pancrelipase USP Grade with each of the following enzymes: Lipase from *Thermomyces lanuginosus* (example 290); Lipase QLM from Meito Sangyo (example 30); and Lipase TL from Meito Sangyo (example 31).

### Example 32: Enzymatic esterification of CMH

A vial (20ml), equipped with magnetic stirrer, was charged with toluene (3ml), vinyl acetate (0.255ml, 2.76mmol), Pancrelipase USP Grade (20mg) and CMH-Racemate (187mg, 0.092mmol). The mixture was stirred for 6 days at room temperature. A sample was taken from the mixture (0.5ml) and dried with N₂ flow. The presence of CMH-ester in the sample was analyzed by HPLC.

### Example 33-35: Enzymatic esterification of CMH

The procedure of example 32 was repeated substituting the Pancrelipase USP Grade with each of the following enzymes: Lipase from *Thermomyces lanuginosus* (example 33); Lipase QLM (example 34); and Lipase TL (example 35).

### Example 36: Enzymatic esterification of CMH

A vial (20ml), equipped with magnetic stirrer, was charged with toluene (3ml), vinyl acetate (0.255ml, 2.76mmol), Lipase from *Thermomyces lanuginosus* (100mg) and CMH-Racemate (187mg, 0.092mmol). The mixture was stirred for 4 days at room temperature. A sample was taken from the mixture (0.5ml) and dried with N₂ flow. The presence of CMH-ester in the sample was analyzed by HPLC.

### Example 37: Enzymatic esterification of CMH

A vial (20ml), equipped with magnetic stirrer, was charged with toluene (3ml), vinyl acetate (0.255ml, 2.76mmol), Lipase from *Thermomyces lanuginosus* (100mg), tetrahydrofuran 98% (0.3ml, 10%) and CMH-Racemate (187mg, 0.092mmol). The resulting mixture was stirred for 4 days at room temperature. A sample was taken from the mixture (0.5ml) and dried with N₂ flow. The presence of CMH-ester in the sample was analyzed by HPLC.

### Example 38: Enzymatic esterification of CMH

The procedure of example 37 was repeated with Lipase from *Thermomyces lanuginosus.*

### Example 39: Enzymatic esterification of CMH

A vial (20ml), equipped with magnetic stirrer, was charged with iso propyl ether 99% (3ml), vinyl acetate (0.255ml, 2.76mmol), Lipase from *Thermomyces lanuginosus* (100mg), and CMH-Racemate (187mg, 0.092mmol). The resulting mixture was stirred for 4 days at room temperature. A sample was taken from the mixture (0.5ml) and dried with N₂ flow. The presence of CMH-ester in the sample was analyzed by HPLC.

### Example 40: Enzymatic esterification of CMH

A vial (20ml), equipped with magnetic stirrer, was charged with methyl *tert*-butyl ether (3ml), vinyl acetate (0.255ml, 2.76mmol), Lipase from *Thermomyces lanuginosus* (100mg), and CMH-Racemate (187mg, 0.092mmol). The resulting mixture was stirred for 4 days at room temperature. A sample was taken from the mixture (0.5ml) and dried with N₂ flow. The presence of CMH-ester in the sample was analyzed by HPLC.

### Example 41: Enzymatic esterification of CMH

A vial (20ml), equipped with magnetic stirrer, was charged with methyl isobutyl ketone (3ml), vinyl acetate (0.255ml, 2.76mmol), Lipase from *Thermomyces lanuginosus* (100mg), and CMH-Racemate (187mg, 0.092mmol). The resulting mixture was stirred for 4 days at room temperature. A sample was taken from the mixture (0.5ml) and dried with N₂ flow. The presence of CMH-ester in the sample was analyzed by HPLC.

### Example 42: Enzymatic esterification of CMH

A vial (20ml), equipped with magnetic stirrer, was charged with toluene (3ml), vinyl acetate (0.255ml, 2.76mmol), Lipase from *Thermomyces lanuginosus* (100mg), tetrahydrofuran 98% (0.6ml, 20%) and CMH-Racemate (187mg, 0.092mmol). The mixture was stirred for 4 days at 37°C. A sample was taken from the mixture (0.5ml) and dried with N₂ flow. The presence of CMH-ester in the sample was analyzed by HPLC.

### Example 43: Enzymatic esterification of CMH

A three necked 50ml flask, equipped with magnetic stirrer, was charged with toluene (4ml), abs EtOH (0.3ml, 5mmol), CAL B (300mg) and CMH-Racemate (187mg, 1mmol). The resulting mixture was stirred for 4 days at 50°C. A sample was taken from the mixture (1ml) and evaporated until dryness. The presence of CMH-ester in the sample was analyzed by HPLC.

### Example 44: Enzymatic esterification of CMH

A vial (20ml), equipped with magnetic stirrer, was charged with toluene (4ml), vinyl acetate (0.25ml, 5mmol), CAL B (144.5mg) and CMH-Racemate (0.1g, 0.535mmol). The resulting mixture was stirred for 4 days at 50°C. A sample was taken from the mixture (1ml) and evaporated until dryness. The presence of CMH-ester in the sample was analyzed by HPLC.

### Example 45: Enzymatic esterification of CMH

A vial (20ml), equipped with magnetic stirrer, was charged with methyl iso butyl ketone (10ml), vinyl acetate (0.46ml, 5mmol), CAL B, stabilized (-400mg), Molecular sieve (3À, ∼100mg) and CMH-Racemate (187mg, 0.647mmol). The mixture was stirred for 47 hrs at 50°C. Sample was taken from the mixture (1ml), the presence of CMH-ester was analyzed by HPLC.

### Example 46: Enzymatic esterification of CMH

A vial (20ml), equipped with magnetic stirrer, was charged with solution of acetone 30% and toluene 70% (10ml), vinyl acetate (0.46ml, 5mmol), CAL B, stabilized (-400mg), Molecular sieve (3À, ∼100mg) and CMH-Racemate (193mg, 1mmol). The mixture was stirred for 47 hrs at 50°C. Sample was taken from the mixture (1ml), the presence of CMH-ester was analyzed by HPLC.

### Example 47: Enzymatic esterification of CMH

A vial (20ml), equipped with magnetic stirrer, was charged with solution of acetone 30% and toluene 70% (10ml), vinyl butyrate (0.46m1,3.6mmole), CAL B, stabilized (-400mg), Molecular sieve (3À, 100mg) and CMH-Racemate (199mg, 1mmol). The mixture was stirred for 47 hrs at 50°C. Sample was taken from the mixture (1ml), the presence of CMH-ester was analyzed by HPLC.

### Example 48: Enzymatic esterification of CMH

A vial (20ml), equipped with magnetic stirrer, was charged with solution of methyl iso butyl ketone (10ml), vinyl butyrate (0.46ml, 3.6mmole), CAL B, stabilized (-400mg), Molecular sieve (3À, 100mg) and CMH-Racemate (189.5mg, 1mmol). The mixture was stirred for 47 hrs at 50°C. Sample was taken from the mixture (1ml), the presence of CMH-ester was analyzed by HPLC.

### Example 49: Enzymatic esterification of CMH

A vial (20ml), equipped with magnetic stirrer, was charged with solution of acetone 70% and toluene 30% (10ml), vinyl acetate (0.46ml, 5mmole), CAL B stabilized enzyme (823.5mg), Molecular sieve (3À, 113mg) and CMH-Racemate (186mg, 1mmol). The mixture was stirred for 64 hrs at ∼50°C. Sample was taken from the mixture (1ml), the presence of CMH-ester was analyzed by HPLC.

### Example 50: Enzymatic esterification of CMH

A vial (20ml), equipped with magnetic stirrer, was charged with solution of acetone 50% and toluene 50% (10ml), vinyl acetate (0.46ml, 5mmole), CAL B stabilized enzyme (802mg), Molecular sieve (3À, 100mg) and CMH-Racemate (201.3mg, 1mmol). The mixture was stirred for 64 hrs at ∼50°C. Sample was taken from the mixture (1ml), the presence of CMH-ester was analyzed by HPLC.

### Example 51: Enzymatic esterification of CMH

A vial (20ml), equipped with magnetic stirrer, was charged with solution of acetone 30% and toluene 70% (10ml), vinyl acetate (0.46ml, 5mmole), TL stabilized enzyme (802.5mg), Molecular sieve (3À, 105.5mg) and CMH-Racemate (194mg, 1mmol). The mixture was stirred for 48 hrs at ∼50°C. Sample was taken from the mixture (1ml), the presence of CMH-ester was analyzed by HPLC.

### Example 52: Enzymatic esterification of CMH

A vial (20ml), equipped with magnetic stirrer, was charged with solution of methyl isobutyl ketone (10ml), Vinyl acetate (0.46ml, 5mmole), TL stabilized enzyme (825mg), Molecular sieve (3À, 107mg) and CMH-Racemate (183mg, 1mmol). The mixture was stirred for 48 hrs at ∼50°C. Sample was taken from the mixture (1ml), the presence of CMH-ester was analyzed by HPLC.

### Example 53: Preparation of (S)-Pregabalin: Example from U.S. Patent No. 5,637,767 (col. 12,1. 46 to col. 13,1. 32)

An 800 1 still was charged with (S)-3-cyano-5-methyl hexanoic acid, ethyl ester (50.1 kg, 273 mol) and ethyl alcohol 2B (53 kg). A solution of potassium hydroxide (17.8 kg, 317 mol) in water (56 1) was added, controlling the addition rate to maintain the batch temperature below 25°C. The mixture was stirred at 20°C to 25°C for about 1.5 hours. The batch was transferred to a hydrogenator containing sponge nickel (15.0 kg, 50% water wet), followed by a rinse of ethyl alcohol 2B (27 kg). The mixture was treated with hydrogen at about 50 psi for about 19 hours (hydrogen uptake stopped).

The nickel was removed by filtration, and the filter cake was rinsed with a mixture of 39 kg ethyl alcohol 2B and 111 1 of water. Glacial acetic acid (22.8 kg, 380 mol) was added to the filtrate, while maintaining the batch temperature at less than 40°C. The batch was heated to 70° to 75°C to dissolve the solids. The batch was slowly cooled to 0°C to 5°C to crystallize the product.

The solid was collected on a centrifuge, and rinsed with 160 1 isopropyl alcohol that was previously cooled to 0° to 5° C.

The damp solid was dried in a vacuum tray drier under vacuum at 35° to 45°C (28 hours) to give (S)-3-aminomethyl-5-methylhexanoic acid.

### Example 54: Conversion of (R)-CMH to (S)-Pregabalin: Example 12 from U.S. Publication No. 2007/0073085

A reactor (0.5 L) was loaded with water (165 ml) and NaOH (35.5 g) to obtain a solution. The solution was cooled to 15°C and (R)--CMH (33 g) was added. Br₂ (28.51 g) was added dropwise (15 min) while keeping the temperature below 25°C. The mixture was heated to 60°C for 15 min and then cooled to 15°C. Iso-butanol was added (100 ml) and then a solution of H₂SO₄ (66%) (33 ml) was added. The phases were separated, and the aqueous phase was extracted with Iso-butanol (83 ml). To the combined organic phases Bu₃N (34.2 g) was added and the mixture was cooled to 2°C, and stirred for 2 hours. The solid was filtered, washed and dried at 55°C under vacuum, providing (S)-PREGABALIN with total purity 99.86% area by HPLC.
The present invention also relates to the following numbered embodiments:
1. A process for preparing a pregabalin intermediate of the following formula I 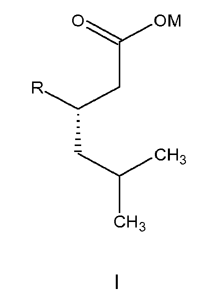 comprising:
   a) combining an ester of the following formula II 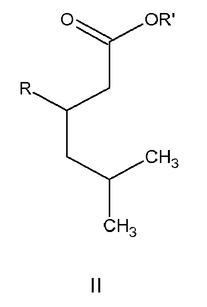 a hydrolase, a buffer, and optionally a base to obtain a mixture; and
   b) maintaining the mixture at a temperature of about 5°C to about 60°C to obtain the pregabalin intermediate of formula I,
   wherein R is CH₂CONR''₂, CH₂CO₂R' or CN; R' is a C₁₋₆ hydrocarbyl; R" is hydrogen or a C₁₋₆ hydrocarbyl; and M is a metal.
2. The process of embodiment 1, wherein R is CN or CH₂CONH₂.
3. The process of embodiment 1 or 2, wherein R' is ethyl or methyl.
4. The process of any one of embodiments 1 to 3, wherein M is an alkali metal.
5. The process of any one of embodiments 1 to 4, wherein the hydrolase is an esterase, protease or lipase.
6. The process of embodiment 5, wherein the esterase is selected from the group consisting of Esterase PF2 recombinant in *E.Coli ,* Esterase BS1 recombinant in *E.Coli ,* Esterase BS2 recombinant in *E.Coli,* Esterase BS2 CLEA recombinant in *E.Coli,* Esterase BS3 recombinant in *E.Coli,* Esterase BS4 recombinant in *E.Coli,* Esterase PL from porcine liver, Esterase SD recombinant in *E.Coli,* Esterase RO, and Esterase TL recombinant in *Aspergillus oryzae.*
7. The process of embodiment 5 or 6, wherein the lipase is selected from the group consisting of Lipase from *Thermomyces lanuginosus,* Lipase P2 from *Pseudomonas cepacia* Lipase PS from *Pseudomonas stutzeri,* Lipase RS from *Rhizopus sp.,* Lipase PF from *Pseudomonas fluorescens,* Lipase PC from *Penicillium camenbertii,* Lipase P1 from *Pseudomonas cepacia,* Lipase AN from *Aspergillus niger,* Lipase A from *Achromobacter sp.,* Lipase AS1 from *Alcaligenes sp.,* Lipase AS2 *Alcaligenes sp,* Lipase C2 from *Candida cylindracea,* Lipase C1 from *Candida cylindracea,* Lipase lipozym TL IM, Lipase lipozym TL 100L, *Candida antarctica* lipase B (CALB), CHIRAZYME E-1 pig liver esterase, Lipase from *Pseudomonas sp.* L-6 , *Candida antarctica* lipase A (CALA), *Candida rugosa* lipase (L-3), Pancreatic lipase USP Grade, Lipase QLM, and Lipase TL.
8. The process of any of embodiment 5 to 7, wherein the protease is chymotrypsin.
9. The process of any of embodiments 5 to 8, wherein the esterase is CALB, CHIRAZYME E-1 pig liver esterase, Esterase BS3 recombinant in *E.Coli,* or Esterase PL from porcine liver.
10. The process of any one of embodiments 1 to 9, wherein the buffer is present in an amount sufficient to provide a pH of about 6 to about 9.
11. The process of any one of embodiments 1 to 10, wherein the base is a hydroxide, carbonate, or hydrogen carbonate of an alkali metal or alkaline earth metal hydroxide.
12. The process of any one of embodiments 1 to 11, wherein the base is sodium hydroxide or potassium hydroxide.
13. The process of any one of embodiments 1 to 12, wherein the hydrolase, the buffer, and optionally the base are combined, followed by addition of the ester of formula II to obtain the mixture.
14. The process of any one of embodiments 1 to 13, wherein a co-solvent is combined with the buffer.
15. The process of embodiment 14, wherein the co-solvent is selected from the group consisting of sulfoxides, amides, alcohols, ketones and nitriles.
16. The process of embodiment 14 or 15, wherein the co-solvent is selected from the group consisting of C₂₋₄ sulfoxides, C₃₋₆ amides, C₁₋₆ alcohols, C₂₋₆ ketones, and C₁₋₅ nitriles.
17. The process of any one of embodiments 14 to 16, wherein the co-solvent is selected from the group consisting of dimethylsulfoxide, dimethylformamide, isopropyl alcohol, acetone, and acetonitrile.
18. The process of any one of embodiments 1 to 17, wherein the mixture is maintained at a temperature of about 20°C to about 27°C to obtain the pregabalin intermediate of formula I.
19. The process of any one of embodiments 1 to 18, wherein R is CN.
20. The process of embodiment 19, wherein the ester of formula II is prepared by decarboxylating a (±)-2-carboxyalkyl-3-cyano-5-methyl hexanoic acid alkyl ester of the following formula 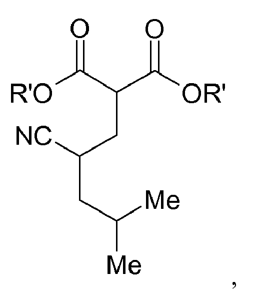 by combining it with an alkaline hydroxide; wherein R' is a C₁₋₆ hydrocarbyl.
21. The process of embodiment 20, wherein R' is ethyl or methyl.
22. The process of embodiment 20 or 21, wherein the alkaline hydroxide is potassium hydroxide.
23. The process of any one of embodiments 20 to 22, wherein the (±)-2-carboxyakyl-3-cyano-5-methyl hexanoic acid alkyl ester and the alkaline hydroxide are combined in the presence of a solvent.
24. The process of embodiment 23, wherein the solvent is selected from the group consisting of water, a polar organic solvent, and mixtures thereof.
25. The process of embodiment 24, wherein the polar organic solvent is a C₁₋₅ alcohol.
26. The process of embodiment 25, wherein the C₁₋₅ alcohol is methanol or ethanol.
27. The process of any one of embodiments 20 to 26, wherein the decarboxylation is done under heating to obtain the ester of formula II.
28. The process of embodiment 27, wherein the heating is to a temperature of about 60°C to about 180°C.
29. A process for preparing a pregabalin intermediate of the following formula I 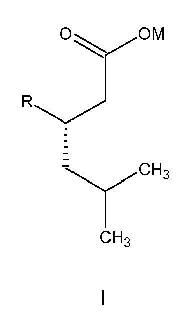 comprising enzymatically hydrolyzing an ester of the following formula II 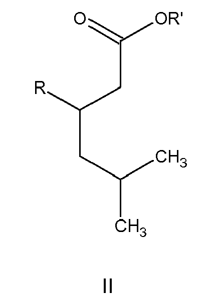 in the presence of a buffer and optionally a base, wherein R is CH₂CONR''₂, CH₂CO₂R' or CN; R' is a C₁₋₆ hydrocarbyl; R" is hydrogen or a C₁₋₆ hydrocarbyl; and M is a metal.
30. A process for preparing a pregabalin intermediate of the following formula I-acid 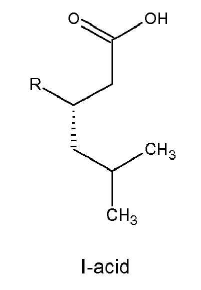 comprising:
   a) preparing a pregabalin intermediate of the following formula I 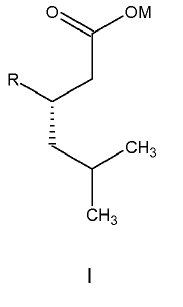 by the process of any one of embodiments 1 to 29; and
   b) converting the pregabalin intermediate of formula I into the pregabalin intermediate of formula I-acid.
31. A process for preparing (S)-pregabalin comprising:
   a) preparing a pregabalin intermediate of the following formula I 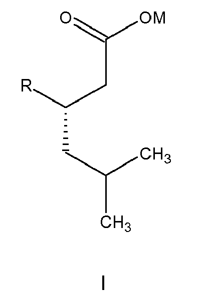 by the process of any one of embodiments 1 to 29; and
   b) converting the pregabalin intermediate of formula I into (S)-pregabalin.

## Claims

1. A process for preparing a pregabalin intermediate of the following formula I-acid 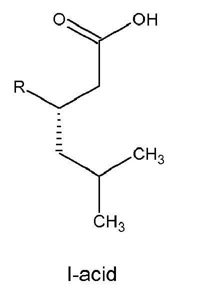 comprising combining a compound of the following formula III, 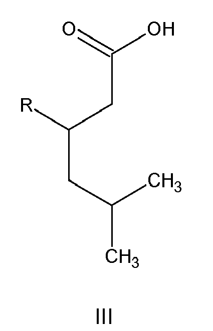 an alcohol or an ester, and an enzyme to obtain the pregabalin intermediate of formula I-acid, wherein R is CH₂CONR''₂, CH₂CO₂R' or CN; R' is a C₁₋₆ hydrocarbyl; and R" is a hydrogen or a C₁₋₆ hydrocarbyl.

2. The process of claim 1, wherein R is CN or CH₂CONH₂.

3. The process of claim 1 or claim 2, wherein the compound of formula III, the alcohol or ester, and the enzyme are combined in the presence of a solvent, preferably wherein the solvent is selected from the group consisting of aromatic hydrocarbons, ethers, ketones, nitriles, chlorinated hydrocarbons, amide, and mixtures thereof.

4. The process of claim 3, wherein the solvent is selected from a group consisting of:
C₆₋₈ aromatic hydrocarbon, C₂₋₈ linear, branched or cyclic ether, C₂₋₈ ketone, C₂₋₅ nitrile, C₁₋₄ chlorinated hydrocarbon, C₃₋₆ amide, and mixtures thereof, preferably wherein the solvent is selected from the group consisting of:
toluene, diisopropylether, methyl-tertbutylether, tetrahydrofuran, methyl-ethyl ketone, methyl-isobutyl ketone, acetone, acetonitrile, dichloromethane, tetrachloromethane, dimethylformamide, and mixtures thereof.

5. The process of claim 3 or claim 4, wherein the solvent is toluene or a mixture of toluene and acetone.

6. The process of any one of claims 1 to 5, wherein the enzyme is a hydrolase, preferably wherein the hydrolase is an esterase, protease or lipase.

7. The process of claim 6, wherein the esterase is selected from the group consisting of Esterase PF2 recombinant in *E.Coli,* Esterase BS1 recombinant in *E.Coli,* Esterase BS2 recombinant in *E. Coli,* Esterase BS2 CLEA recombinant in *E. Coli,* Esterase BS3 recombinant in *E.Coli,* Esterase BS4 recombinant in *E.Coli,* Esterase PL from porcine liver, Esterase SD recombinant in *E. Coli,* Esterase RO, and Esterase TL recombinant in *Aspergillus oryzae,* preferably wherein the esterase is CALB, CHIRAZYME E-1 pig liver esterase, Esterase BS3 recombinant in *E.Coli,* or Esterase PL from porcine liver.

8. The process of claim 6, wherein the lipase is selected from the group consisting of Lipase from *Thermomyces lanuginosus,* Lipase P2 from *Pseudomonas cepacia* Lipase PS from *Pseudomonas stutzeri,* Lipase RS from *Rhizopus sp.,* Lipase PF from *Pseudomonas fluorescens,* Lipase PC from *Penicillium camenbertii,* Lipase P1 from *Pseudomonas cepacia,* Lipase AN from *Aspergillus niger,* Lipase A from *Achromobacter sp.,* Lipase AS1 from *Alcaligenes sp.,* Lipase AS2 *Alcaligenes sp,* Lipase C2 from *Candida cylindracea,* Lipase C from *Candida cylindracea,* Lipase lipozym TL IM, Lipase lipozym TL 100L, *Candida antarctica* lipase B (CALB), CHIRAZYME E-1 pig liver esterase, Lipase from *Pseudomonas sp.* L-6 , *Candida antarctica* lipase A (CALA), *Candida rugosa* lipase (L-3), Pancreatic lipase USP Grade, Lipase QLM, and Lipase TL.

9. The process of claim 6, wherein the protease is chymotrypsin.

10. The process of any one of claims 1 to 9, wherein the alcohol is selected from methanol, ethanol, propanol, n-butanol, and mixtures thereof.

11. The process of any one of claims 1 to 10, wherein the ester is vinyl acetate or vinyl butyrate.

12. The process of any one of claims 1 to 11, wherein the combination of the compound of formula III, the alcohol or ester, and the enzyme is maintained at a temperature of about 5°C to about 50°C to obtain the pregabalin intermediate of formula I-acid.

13. The process of any one of claims 1 to 12, wherein the ester or alcohol and the compound of formula III are combined in a ratio of about 1 mole of ester or alcohol to about 1 mole of the compound of formula III.

14. The process of any one of claims 1 to 12, wherein the ester or alcohol and the compound of formula III are combined in a ratio of greater than about 1 mole of the ester or alcohol to about 1 mole of the compound of formula III, preferably wherein the ester or alcohol and the compound of formula III are combined in a ratio of about 3 to about 10 moles of the ester or alcohol to about 1 mole of the compound of formula III.

15. A process for preparing a pregabalin intermediate of the following formula I-acid 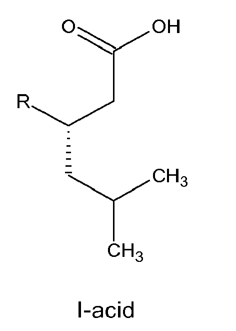 comprising enzymatically esterifying a compound of the following formula III, 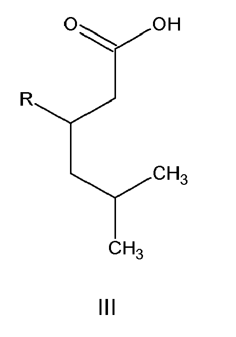 wherein R is CH₂CONR"₂, CH₂CO₂R' or CN; R' is a C₁₋₆ hydrocarbyl; and R" is a hydrogen or a C₁₋₆ hydrocarbyl.

16. A process for preparing (S)-pregabalin comprising:
a) preparing a pregabalin intermediate of the following formula I-acid 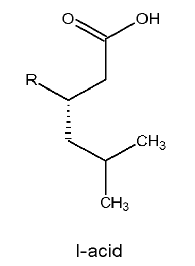 by the process of any one of claims 1 to 15; and
b) converting the pregabalin intermediate of formula I-acid into (S)-pregabalin.
